# FASCICULE DE BREVET EUROPEEN

(11) **EP 0 427 625 B1**
(45) Date de publication et mention de la délivrance du brevet: **04.01.1995**
(21) Numéro de dépôt: 90403161.4
(22) Date de dépôt: 07.11.1990
(51) Int. Cl.: C07D 239/48, A61K 7/06, A61K 31/505

(54) **Nouveaux sels internes des hydroxydes de diamino-2,4 alcoxy-6 sulfooxy-3 pyrimidinium et leur utilisation pour le traitement et la prévention de la chute des cheveux**
Interne Salze des 2,4-Diamino-6-alkoxy-3-sulphooxypyrmidium-Hydroxids und ihre Verwendung für die Behandlung und die Verhütung des Haarausfalls
Internal salts of 2,4-diamino-6-alkoxy-3-sulphooxypyrmidium hydroxide and their use in the treatment and prevention of hair-loss

(30) Priorité: 08.11.1989 FR 8914667
(43) Date de publication de la demande: 15.05.1991
(73) Titulaire: L'OREAL, 75008 Paris (FR)
(72) Inventeur: Gaetani, Quintino, F-93270 Sevran (FR); Estradier, Françoise, F-75018 Paris (FR); Hocquaux, Michel, F-75012 Paris (FR)
(74) Mandataire: Casalonga, Axel

(56) Documents cités:
- WO-A-86/04231
- US-A- 3 382 248

## Description

La présente invention est relative à des nouveaux sels internes des hydroxydes de diamino-2,4 alcoxy-6 sulfooxy-3 pyrimidinium et leurs dérivés, à leur préparation et des compositions cosmétiques ou pharmaceutiques destinées notamment à être utilisées en application topique dans le traitement et la prévention de la chute des cheveux.

On connaît déjà, notamment par l'EP-A-0210 227 le sel interne de l'hydroxyde de pipéridino-6 diamino-2,4 sulfooxy-3 pyrimidinium ou "Sulfate de Minoxidil" pour son utilisation dans le traitement de la chute des cheveux, de la pelade, de la dermatite desquamante, de l'alopécie, etc.

La demanderesse vient de découvrir de nouveaux produits qui sont des sels internes des hydroxydes de diamino-2,4 sulfooxy-3 pyrimidinium, substitués en position 6.

Elle a découvert que ces produits étaient particulièrement efficaces dans le traitement de la repousse des cheveux, en particulier pour induire et stimuler la croissance des cheveux et freiner leur chute et pouvaient être utilisés, notamment, dans le traitement des maladies du cuir chevelu, telles que la pelade, la dermatite desquamante, l'alopécie.

L'invention a donc pour objet des sels internes des hydroxydes de diamino-2,4 sulfooxy-3 pyrimidinium substitués en position 6 et leurs dérivés.

Un autre objet de l'invention est constitué par leur procédé de préparation.

L'invention concerne également des compositions cosmétiques et/ou pharmaceutiques mettant en oeuvre ces composés.

D'autres objets de l'invention apparaîtront à la lecture de la description et des exemples qui suivent.

Les composés conformes à l'invention sont essentiellement caractérisés par le fait qu'ils répondent à la formule : 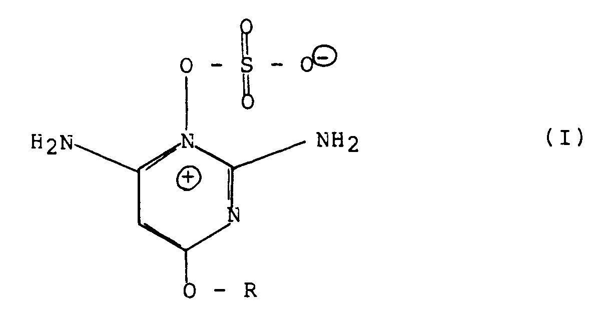
dans laquelle :
R désigne un radical alkyle, linéaire ou ramifié en C₁-C₁₈, alcényle en C₂-C₁₈, un radical cyclique, saturé ou insaturé ou un radical alkyle en C₁-C₆ portant un noyau aromatique ou hétérocyclique, tel que la pyridine.

Les composés plus particulièrement préférés, conformes à l'invention, sont les composés dans lesquels le groupement alkyle désigne un groupement ayant 2 à 12 atomes de carbone, le noyau aromatique désigne de préférence un groupement phényle.

Les composés préférés définis ci-dessus, sont ceux dans lesquels R est choisi parmi les groupements éthyle, butyle, isobutyle, octyle, lauryle, éthyl-2 hexyle, hexényle, diméthyl-3,3 butyle, undécényle, cyclohexyle, phénéthyle ou benzyle.

Les composés particulièrement préférés de l'invention sont constitués par les sels internes des hydroxydes de diamino-2,4 (n-butyloxy)-6 sulfooxy-3 pyrimidinium, de diamino-2,4 éthyloxy-6 sulfooxy-3 pyrimidinium, de diamino-2,4 hexyloxy-6 sulfooxy-3 pyrimidinium.

Les composés conformes à l'invention peuvent être préparés suivant un procédé consistant à faire réagir un composé de formule (II) : 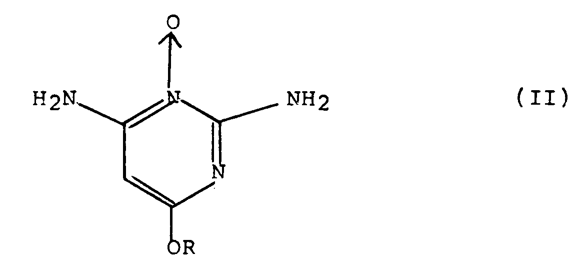
avec un dérivé générateur d'anhydride sulfurique, dans un solvant polaire, tel que le diméthylformamide ou le chloroforme, à des températures comprises entre 0°C et 80°C.

Comme générateurs d'anhydride sulfurique, on utilise, de préférence, un complexe de SO₃ et d'amine tertiaire ou un mélange formé in situ, d'acide chlorosulfonique et d'amine tertiaire stériquement encombrée, telle que la N,N-diisopropyléthylamine.

Les composés conformes à l'invention peuvent être utilisés dans le domaine cosmétique ou pharmaceutique, notamment dans les applications topiques, et plus particulièrement dans le traitement ou la prévention de la chute des cheveux, et notamment de la pelade, de l'alopécie, ainsi que des dermatites desquamantes.

Ces compositions sont essentiellement caractérisées par le fait qu'elles contiennent dans un milieu physiologiquement acceptable, approprié pour une application topique, au moins un composé répondant à la formule (I).

Ces compositions peuvent comporter à titre de milieu physiologiquement acceptable, tout milieu approprié pour l'application topique, soit en cosmétique, soit en pharmacie, et qui soit compatible avec la substance active.

Les composés conformes à l'invention peuvent se trouver dans ce milieu, soit à l'état dissous, soit à l'état dispersé, notamment sous forme micronisée.

Les compositions destinées à être utilisées dans le domaine pharmaceutique se présentent sous forme d'onguent, de teinture, de crème, de pommade, de poudre, de timbre, de tampon imbibé, de solution, d'émulsion ou d'émulsion vésiculaire, de mousse, de lotion, de gel, de spray ou de suspension. Elles peuvent être, soit anhydres, soit aqueuses, selon l'indication clinique.

Les composés sont présents dans ces compositions pharmaceutiques à des concentrations comprises entre 0,1 et 10% en poids, et en particulier comprises entre 0,2 et 5% en poids.

Les compositions cosmétiques sont notamment destinées à être utilisées sous forme de lotion, de gel, de crème, de savon, de shampooing, d'aérosol ou de mousse et contiennent, dans un support physiologiquement acceptable, au moins un composé de formule (I) ou l'un de ses sels.

La concentration des composés de formule (I) dans ces compositions est, de préférence, comprise entre 0,01 et 3% en poids et en particulier entre 0,05 et 2% en poids.

Les compositions conformes à l'invention peuvent contenir différents additifs habituellement utilisés en cosmétique ou en pharmacie et en particulier des substances actives, telles que des agents hydratants comme la thiamorpholinone et ses dérivés ou l'urée; des agents antiséborrhéiques, tels que la S-carboxyméthylcystéine, la S-benzylcystéamine et leurs dérivés; la tioxolone;

Les composés conformes à l'invention peuvent être associés à des composés améliorant encore leur activité sur la repousse et/ou sur le freinage de la chute des cheveux, tels que les composés suivants :
- les esters d'acide nicotinique, dont les nicotinates d'alkyle en C₁-C₆ et notamment le nicotinate de méthyle;
- les agents anti-inflammatoires stéroïdiens et non stéroïdiens bien connus dans l'état de la technique et en particulier l'hydrocortisone, ses sels et ses dérivés, l'acide niflumique;
- les rétinoïdes comme l'acide t-trans rétinoïque appelé encore trétinoïne, l'isotrétinoïne, le rétinol ou vitamine A et ses dérivés, tels que l'acétate, le palmitate ou le propionate, le motrétinide, l'étrétinate, le t-trans rétinoate de zinc;
- les agents antibactériens choisis parmi les macrolides, les pyranosides et les tétracyclines et notamment l'érythromycine;
- les agents antagonistes de calcium, tels que la cinnarizine et le diltiazem;
- les hormones, telles que l'estriol ou des analogues ou la thyroxine et ses sels;
- les agents antiandrogènes, tels que l'oxendolone, la spironolactone, le diéthylstilbestrol;
- les capteurs de radicaux OH, tels que le diméthylsulfoxyde.

On peut également associer avec les composés de l'invention des composés tels que le diazoxyde correspondant au méthyl-3 chloro-7-2H-benzothiadiazine-1,2,4-dioxyde-1,1; la spiroxasone ou 7 α-(acétylthio)-4′,5′-dihydrospiro-7α[androst 4-ène-17,2′-(3′H)furan]-3 one; les phospholipides, tels que la lécithine; les acides linoléique et linolénique; l'acide salicylique et ses dérivés décrits dans le brevet français 2 581 542, et plus particulièrement les dérivés d'acide salicylique porteurs d'un groupement alcanoyle ayant 2 à 12 atomes de carbone en position 5 du cycle benzénique; les acides hydroxycarboxyliques ou cétocarboxyliques et leurs esters, les lactones et leurs sels correspondants; l'anthraline ou le trihydroxy-1,8,9 anthracène, les caroténoïdes, les acides eicosatétraynoïque-5,8,11,14 et eicosatriynoïque-5,8,11, leurs esters et amides.

Ces compositions peuvent également contenir des agents conservateurs, des agents stabilisants, des agents régulateurs de pH, des agents modificateurs de pression osmotique, des agents tensio-actifs, des filtres UV-A et UV-B, des agents antioxydants, tels que l' α-tocophérol, le butylhydroxyanisole, le butylhydroxytoluène.

Le milieu physiologiquement acceptable peut être constitué par de l'eau ou un mélange d'eau et d'un solvant ou un mélange de solvants, les solvants étant choisis parmi les solvants organiques acceptables sur le plan cosmétique ou pharmaceutique. Ce sont notamment les alcools inférieurs en C₁-C₄, comme l'alcool éthylique, l'alcool isopropylique, l'alcool tertiobutylique, les alkylèneglycols, les alkyléthers d'alkylèneglycol et de dialkylèneglycol, tels que le monoéthyléther d'éthylèneglycol, le monométhyléther de propylèneglycol, le monométhyléther de diéthylèneglycol. Les solvants, lorsqu'ils sont présents, le sont dans des proportions comprises entre 1 et 80% en poids par rapport au poids total de la composition.

Les milieux physiologiquement acceptables peuvent être épaissis à l'aide d'agents épaississants et/ou gélifiants habituellement utilisés en cosmétique ou dans le domaine pharmaceutique. On peut plus particulièrement citer les hétérobiopolysaccharides, tels que la gomme de xanthane, les scléroglucanes, les dérivés de cellulose comme les éthers de cellulose, le polymères acryliques, réticulés ou non.

Les épaississants sont présents de préférence dans des proportions comprises entre 0,1 et 5% en poids et en particulier entre 0,4 et 3% en poids par rapport au poids total de la composition.

Un autre objet de l'invention est constitué par l'utilisation de la composition définie ci-dessus, pour la préparation d'un médicament ayant pour effet d'induire ou de stimuler la croissance des cheveux et de freiner leur chute.

Le traitement thérapeutique consiste principalement à appliquer sur les zones alopéciques du cuir chevelu d'un individu, la composition telle que définie ci-dessus.

Le mode d'application préféré consiste à appliquer 1 à 2 g de la composition sur la zone alopécique, à une fréquence de une à deux applications par jour, pendant 1 à 7 jours par semaine et ceci pendant une durée de 1 à 6 mois.

Les compositions peuvent notamment être utilisés dans le traitement de la pelade, de l'alopécie, de la dermatite desquamante.

Les exemples suivants sont destinés à illustrer l'invention.

### EXEMPLE 1

### Préparation du sel interne de l'hydroxyde de diamino-2,4 éthoxy-6 sulfooxy-3 pyrimidinium.

A une solution de 2,05 ml (0,012 mole) de N,N-diisopropyléthylamine dans 15 ml de chloroforme refroidie dans la glace, on ajoute 0,4 ml (0,006 mole) d'acide chlorosulfonique.

Après 30 minutes de réaction, on ajoute 0,51 g (0,003 mole) de diamino-2,4 éthoxy-6 pyrimidine oxyde-3 et on maintient 4 heures à température ambiante, sous azote.

Après évaporation du solvant, le résidu est repris dans l'eau et filtré après cristallisation d'un produit blanc.

Le sulfate ainsi obtenu est recristallisé dans un mélange DMF/eau et conduit, après filtration et séchage, à 0,3 g de sulfate interne, soit un rendement de 40%.

Le produit obtenu fond à 205°C avec décomposition et présente en spectroscopie de masse un pic moléculaire conforme de 250.
Formule brute : C₆H₁₀N₄O₅S

### EXEMPLE 2

### Préparation du sel interne de l'hydroxyde de diamino-2,4 (n-butyloxy)-6 sulfooxy-3 pyrimidinium.

On procède comme dans l'exemple 1, en faisant réagir, durant 2 heures, 0,59 g de diamino-2,4 (n-butyloxy)-6 pyrimidine oxyde-3.

Après recristallisation dans DMF/eau, on obtient, avec 60% de rendement, un composé fondant à 139°C avec décomposition.

| Analyse élémentaire pour C₈H₁₄N₄O₅S | | | | | |
|---|---|---|---|---|---|
| | C | H | N | O | S |
| Calculé | 34,53 | 5,03 | 20,14 | 28,77 | 11,53 |
| Trouvé | 34,59 | 5,12 | 20,19 | 28,66 | 11,70 |

La spectroscopie de RMN du proton et la spectroscopie de masse confirment la structure du sulfate interne attendu.
PM = 278.

### EXEMPLE 3

### Préparation du sel interne de l'hydroxyde de diamino-2,4 (n-octyloxy)-6 sulfooxy-3 pyrimidinium.

On procède selon l'exemple 1 avec 0,76 g de diamino-2,4 (n-octyloxy)-6 pyrimidine oxyde-3 en maintenant le milieu réactionnel durant 2 heures entre 0 et 5°C.

Le produit obtenu est recristallisé dans un mélange acétonitrile-eau. On obtient avec 78% de rendement un sulfate interne cristallisé avec 1/2 molécule d'eau et se décomposant à 167°C.

| Analyse élémentaire du semi-hydrate pour C₁₂H₂₂N₄O₅S; 1/2 H₂O | | | | | |
|---|---|---|---|---|---|
| | C | H | N | O | S |
| Calculé | 41,98 | 6,70 | 16,33 | 25,66 | 9,33 |
| Trouvé | 42,31 | 6,61 | 16,39 | 25,44 | 9,08 |

La spectroscopie de RMN du proton et la spectroscopie de masse confirment la structure attendue.
PM du sulfate semi-hydrate = 343.

### EXEMPLE 4

### Préparation du sel interne de l'hydroxyde de diamino-2,4 (diméthyl-3,3 butyloxy)-6 sulfooxy-3 pyrimidinium.

On fait réagir, selon l'exemple 1, 0,68 g de diamino-2,4 (diméthyl-3,3 butyloxy)-6 pyrimidine oxyde-3 durant 3 heures entre 0 et 5°C.

Après recristallisation du sulfate brut dans un mélange acétonitrile-eau, on obtient, avec 25% de rendement, un composé fondant avec décomposition à 212°C.

La RMN du proton et la spectroscopie de masse confirment la structure attendue.
Formule brute : C₁₀H₁₈N₄O₅S
PM = 306

### EXEMPLE 5

### Préparation du sel interne de l'hydroxyde de diamino-2,4 (hexényle-5 oxy)-6 sulfooxy-3 pyrimidinium.

On fait réagir, selon l'exemple 1, 0,67 g de diamino-2,4 (hexényl-5 oxy)-6 pyrimidine oxyde-3 durant 2 heures 30 minutes entre 5 et 10°C.

Le sulfate brut est recristallisé dans un mélange DMF-eau. On obtient, avec 64% de rendement, un sulfate interne fondant à 147°C avec décomposition.

La RMN du proton et la spectroscopie de masse confirment la structure attendue.
Formule brute : C₁₀H₁₆N₄O₅S
PM = 304.

### EXEMPLE 6

### Préparation du sel interne de l'hydroxyde de diamino-2,4 (undécényl-10 oxy)-6 sulfooxy-3 pyrimidinium.

On procède, selon l'exemple 1, avec 0,88 g de diamino-2,4 (undécényl-10 oxy)-6 pyrimidine oxyde-3 durant 2 heures à une température comprise entre 0 et 5°C.

Le produit brut est recristallisé dans un mélange DMF-eau et conduit, avec 45% de rendement, à un sulfate interne fondant avec décomposition à 137°C.

La RMN du proton et la spectroscopie de masse confirment la structure attendue.
Formule brute : C₁₅H₂₆N₄O₅S
PM = 374.

### EXEMPLE 7

### Préparation du sel interne de l'hydroxyde de diamino-2,4 (phényl-2 éthyloxy)-6 sulfooxy-3 pyrimidinium.

On fait réagir, selon l'exemple 1, 0,74 g de diamino-2,4 (phényl-2 éthyloxy)-6 pyrimidine oxyde-3 durant 1 heure à 10°C.

Après recristallisation dans un mélange acétonitrile-eau, on obtient, avec 40% de rendement, un sulfate interne comportant une 1/2 molécule d'eau et présentant une fusion pâteuse à 115-120°C.

| Analyse élémentaire du semi-hydrate : C₁₂H₁₄N₄O₅S; 1/2 H₂O | | | | | |
|---|---|---|---|---|---|
| | C | H | N | O | S |
| Calculé | 42,98 | 4,48 | 16,71 | 26,27 | 9,55 |
| Trouvé | 43,28 | 4,41 | 16,69 | 25,89 | 9,53 |

La RMN du proton ainsi que la spectroscopie de masse confirment la structure moléculaire attendue.
PM du sulfate semi-hydrate = 335.

### EXEMPLE 8

### Préparation du sel interne de l'hydroxyde de diamino-2,4 héxyloxy-6 sulfooxy-3 pyrimidinium.

On procède comme dans l'exemple 1, en faisant réagir, durant 3 heures 15 minutes entre 0 et 5°C, 0,226 g de diamino-2,4 héxyloxy-6 pyrimidine oxyde-3.

Après recristallisation dans un mélange acétonitrile-eau : 1/1, on obtient le sulfate avec un rendement de 59%.

| Analyse élémentaire de : C₁₀H₁₈N₄O₅S; 1,8 H₂O | | | | | |
|---|---|---|---|---|---|
| | C | H | N | O | S |
| Calculé | 35,46 | 6,38 | 16,54 | 32,15 | 9,46 |
| Trouvé | 35,54 | 6,11 | 16,78 | 31,71 | 9,73 |

La spectroscopie de RMN du proton et la spectroscopie de masse confirment la structure du sulfate interne attendu.
PM = 306 (1,8 H₂O).

### Exemple de composition 1

On prépare un shampooing destiné au traitement de la chute des cheveux, de composition suivante :

| | |
|---|---|
| - Hydroxyde de diamino-2,4 n-butyloxy-6 sulfooxy-3 pyrimidinium | 0,2 g |
| - Tensio-actif non-ionique obtenu par condensation de 3,5 moles de glycidol sur un α-diol en C₁-C₁₄ selon le brevet français n° 71 17206 | 13,0 gMA |
| - Complexant | 0,2 g |
| - Conservateurs | 0,5 g |
| - Eau | q.s.p. 100,0 g |

Cette composition est appliquée sur la chevelure mouillée où on la fait mousser. Après une pose durant quelques minutes à une demi-heure, on rince à l'eau tiède.

### Exemple de composition 2

On prépare une lotion de composition suivante :

| | |
|---|---|
| - Hydroxyde de diamino-2,4 n-butyloxy-6 sulfooxy-3 pyrimidinium | 1,0 g |
| - Ethanol | 50,0 g |
| - Eau | q.s.p. 100,0 g |

### Exemple de composition 3

On prépare une lotion de composition suivante :

| | |
|---|---|
| - Hydroxyde de diamino-2,4 n-butyloxy-6 sulfooxy-3 pyrimidinium | 2,0 g |
| - Propylèneglycol | 22,8 g |
| - Ethanol | 55,1 g |
| - Eau | q.s.p. 100,0 g |

### Exemple de composition 4

On prépare une lotion de composition suivante :

| | |
|---|---|
| - Hydroxyde de diamino-2,4 hexyloxy-6 sulfooxy-3 pyrimidinium | 0,5 g |
| - Propylèneglycol | 6,45 g |
| - Ethanol absolu | q.s.p. 100,0 g |

### Exemple de composition 5

On prépare une lotion de composition suivante :

| | |
|---|---|
| - Hydroxyde de diamino-2,4 (phényl-2 éthyloxy)-6 sulfooxy-3 pyrimidinium | 0,5 g |
| - Ethoxy-2 éthanol | 50,0 g |
| - Ethanol | q.s.p. 100,0 g |

1 à 2 ml des lotions selon les exemples de composition n°2 à 5 sont appliqués sur les zones alopéciques du cuir chevelu ; ces applications, éventuellement accompagnées par un massage pour favoriser la pénétration, étant effectuées une ou deux fois par jour.

## Revendications

1. Composé caractérisé par le fait qu'il répond à la formule : 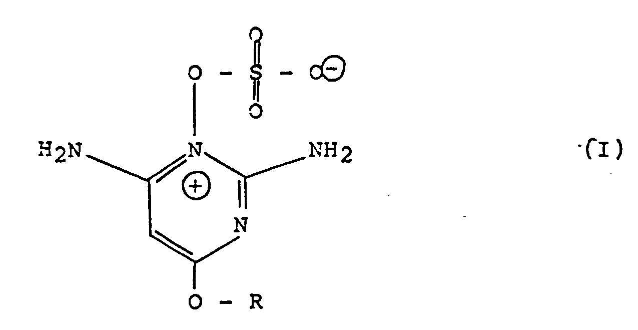 dans laquelle :
R désigne un radical alkyle, linéaire ou ramifié en C₁-C₁₈, alcényle en C₂-C₁₈, un radical cyclique, saturé ou insaturé ou un radical alkyle an C₁-C₆ portant un noyau aromatique ou hétérocyclique.

2. Composé salon la revendication 1, caractérisé par la fait que la noyau hétérocyclique est la pyridine.

3. Composé selon la revendication 1 ou 2, caractérisé par la fait que la radical R dans la formula (I) est choisi parmi las groupements éthyle, butyle, isobutyle, diméthyl-3,3 butyle, octyle, lauryle, éthyl-2 hexyle, hexényle, undécényle, cyclohexyle, phénéthyle ou benzyle.

4. Composé salon l'une quelconque des revendications 1 à 4, caractérisé par la fait que le composé de formule (I) est choisi parmi las sels internes de l'hydroxyde de diamino-2,4 (n-butyloxy)-6 sulfooxy-3 pyrimidinium, de diamino-2,4 éthyloxy-6 sulfooxy-3 pyrimidinium et de diamino-2,4 hexyloxy-6 sulfooxy-3 pyrimidinium.

5. Procédé de préparation des composés selon l'une quelconque des revendications 1 à 4, caractérisé par le fait qu'on fait réagir un composé de formule (II) : 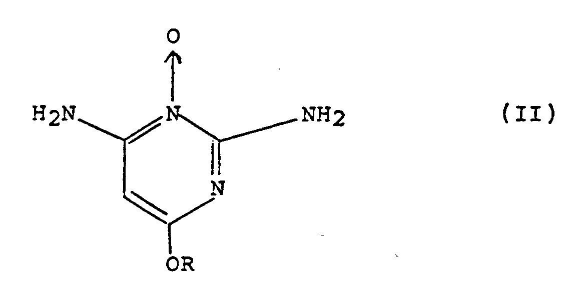 avec un dérivé générateur d'anhydride sulfurique dans un solvant polaire, à une température comprise entre 0°C et 80°C.

6. Procédé selon la revendication 5, caractérisé par le fait que le dérivé générateur d'anhydride sulfurique est choisi parmi les complexes de SO₃ et d'amine tertiaire ou un mélange formé in situ, d'acide chlorosulfonique et d'amine tertiaire stériquement encombrée et que le solvant polaire est constitué par le diméthylformanide ou le chloroforme.

7. Composition destinée à être utilisée en application topique, caractérisée par le fait qu'elle contient dans un milieu physiologiquement acceptable, au moins un composé tel que défini dans l'une quelconque des revendications 1 à 4.

8. Composition salon la revendication 7, caractérisée par le fait qu'elle se présente sous forme d'onguent, de teinture, de crème, de pommade, de poudre, de timbre, de tampon imbibé, de solution, d'émulsion ou d'émulsion vésiculaire, de mousse, de lotion, de gel, de spray ou de suspension anhydre ou aqueuse, en vue de son application pharmaceutique et qu'elle contient au moins un composé tel que défini dans l'une quelconque des revendications 1 à 4.

9. Composition selon la revendication 7 ou 8, caractérisée par le fait que les composés de formula (I) sont présents dans des concentrations comprises entre 0,1 et 10% en poids par rapport au poids total de la composition et en particulier entre 0,2 et 5% en poids.

10. Composition destinée à être utilisée en cosmétique, telle que définie dans la revendication 7; caractérisée par le fait qu'elle se présente sous forme de lotion, de gel, de crème, de savon, de shampooing, d'aérosol ou de mousse et qu'elle contient dans un support acceptable sur le plan cosmétique, au moins un composé tel que défini dans l'une quelconque des revendications 1 à 4, à une concentration comprise entre 0,01 et 3% en poids.

11. Composition selon l'une quelconque des revendications 7 à 10, caractérisée par le fait qu'elle contient en plus des agents hydratants, des agents antiséborrhéiques.

12. Composition selon l'une quelconque des revendications 7 à 11, caractérisée par le fait qu'elle contient également des agents améliorant l'activité des composés de formule (I) au niveau de la repousse ou du freinage de la chute des cheveux.

13. Composition selon la revendication 12, caractérisée par le fait que les agents améliorant l'activité de la repousse et/ou du freinage de la chute des cheveux, sont des esters d'acide nicotinique et des agents anti-inflammatoires stéroïdiens ou non stéroïdiens, des rétinoïdes, des agents anti-bactériens, des agents antagonistes de calcium, des hormones, des agents anti-androgènes, des capteurs de radicaux OH.

14. Composition selon la revendication 12, caractérisée par le fait qu'elle contient à titre de composés améliorant l'activité sur la repousse des cheveux ou le freinage de la chute des cheveux, des composés choisis parmi le diazoxide, la spiroxasone, les phospholipides, les acides linolénique ou linoléique, l'acide salicylique et ses dérivés, des acides hydroxycarboxyliques ou cétocarboxyliques, leurs esters, les lactones et leurs sels correspondants, l'anthraline ou le trihydroxy-1,8,9 anthracène, les caroténoïdes, les acides eicosatétraynoïque-5,8,11,14, eicosatriynoïque-5,8,11, leurs esters et amides.

15. Composition selon l'une quelconque des revendications 7 à 14, caractérisée par le fait que le milieu physiologiquement acceptable est constitué par de l'eau, un mélange d'eau et d'un ou plusieurs solvants organiques ou par un mélange de solvants organiques, les solvants organiques étant pharmaceutiquement ou cosmétiquement acceptables.

16. Composition selon la revendication 15, caractérisée par le fait que les solvants sont choisis parmi les alcools inférieurs en C₁-C₄, les alkylène glycols, les alkyléthers de mono- et de dialkylèneglycol.

17. Composition selon l'une, quelconque des revendications 7 à 16, caractérisée par le fait que le milieu physiologiquement acceptable est épaissi au moyen d'agents épaississants et/ou gélifiants et contient des agents conservateurs, des agents stabilisants, des agents régulateurs de pH, des agents modificateurs de pression osmotique, des agents tensio-actifs, des filtres UV-A et UV-B, des agents antioxydants.

18. Composition selon l'une quelconque des revendications 7 à 17, pour son application comme médicament destiné à être utilisé dans le traitement thérapeutique de la chute des cheveux et pour stimuler leur repousse.

19. Utilisation de la composition telle que définie dans l'une quelconque des revendications 7 à 17, pour la préparation d'un médicament destiné à être utilisé dans le traitament thérapeutique de la chute des cheveux et pour favoriser leur repousse.

## Claims

1. Compound, characterized in that it corresponds to the formula: 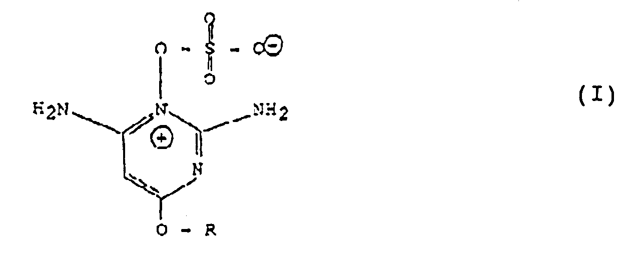 in which:
R denotes a straight-chain or branched C₁-C₁₈ alkyl radical, a C₂-C₁₈ alkenyl radical, a saturated or unsaturated cyclic radical or a C₁-C₆ alkyl radical carrying an aromatic or heterocyclic ring.

2. Compound according to Claim 1, characterized in that the heterocyclic ring is pyridine.

3. Composition according to Claim 1 or 2, characterized in that the radical R in formula (I) is chosen from the ethyl, butyl, isobutyl, 3,3-dimethylbutyl, octyl, lauryl, 2-ethylhexyl, hexenyl, undecenyl, cyclohexyl, phenethyl or benzyl groups.

4. Compound according to any one of Claims 1 to 4, characterized in that the compound of formula (I) is chosen from 2,4-diamino-6-(n-butoxy)-3-sulphooxy-pyrimidinium hydroxide inner salts, 2,4-diamino-6-ethoxy-3-sulphooxy-pyrimidinium hydroxide inner salts and 2,4-diamino-6-hexyloxy-3-sulphooxy-pyrimidinium hydroxide inner salts.

5. Process for the preparation of the compounds according to any one of Claims 1 to 4, characterized in that a compound of formula (II): 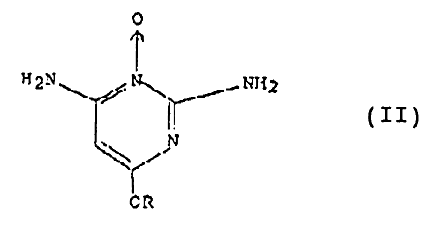 is reacted with a derivative generating sulphur trioxide, in a polar solvent, at a temperature of between 0°C and 80°C.

6. Process according to Claim 5, characterized in that the derivative generating sulphur trioxide is chosen from the complexes of SO₃ and tertiary amine or a mixture, formed in situ, of chlorosulphonic acid and sterically hindered tertiary amine, and in that the polar solvent is dimethylformamide or chloroform.

7. Composition intended to be used by topical application, characterized in that it contains, in a physiologically acceptable medium, at least one compound as defined in any one of Claims 1 to 4.

8. Composition according to Claim 7, characterized in that it is in the form of an unguent, tincture, cream, ointment, powder, patch, impregnated buffer, solution, emulsion or vesicular emulsion, foam, lotion, gel, spray or anhydrous or aqueous suspension, with a view to its pharmaceutical application, and in that it contains at least one compound as defined in any one of Claims 1 to 4.

9. Composition according to Claim 7 or 8, characterized in that the compounds of formula (I) are present in concentrations of between 0.1 and 10% by weight relative to the total weight of the composition, and in particular of between 0.2 and 5% by weight.

10. Composition intended to be used cosmetically, as defined in Claim 7, characterized in that it is in the form of a lotion, gel, cream, soap, shampoo, aerosol or foam, and in that it contains, in a carrier acceptable in the cosmetic field, at least one compound as defined in any one of Claims 1 to 4, at a concentration of between 0.01 and 3% by weight.

11. Composition according to any one of Claims 7 to 10, characterized in that it also contains hydrating agents and antiseborrhoeic agents.

12. Composition according to any one of Claims 7 to 11, characterized in that it also contains agents which improve the activity of the compounds of formula (I) with respect to hair growth or curbing hair loss.

13. Composition according to Claim 12, characterized in that the agents which improve the activity in respect of hair growth and/or the curbing of hair loss are nicotinic acid esters and steroid or non-steroid anti-inflammatory agents, retinoids, anti-bacterial agents, calcium antagonists, hormones, antiandrogens or agents which capture OH radicals.

14. Composition according to Claim 12, characterized in that it contains, as compounds which improve the activity in respect of hair growth or curbing of hair loss, compounds chosen from diazoxide, spiroxasone, phospholipids, linolenic or linoleic acids, salicylic acid and its derivatives, hydroxycarboxylic or ketocarboxylic acids, their esters, the lactones and their corresponding salts, anthralin or 1,8,9-trihydroxyanthracene, carotenoids, 5,8,11,14-eicosatetraynoic and 5,8,11-eicosatriynoic acids and their esters and amides.

15. Composition according to any one of Claims 7 to 14, characterized in that the physiologically acceptable medium is water, a mixture of water and one or more organic solvents or a mixture of organic solvents, the organic solvents being pharmaceutically or cosmetically acceptable.

16. Composition according to Claim 15, characterized in that the solvents are chosen from C₁-C₄ lower alcohols, alkylene glycols and mono- and di-alkylene glycol alkyl ethers.

17. Composition according to any one of Claims 7 to 16, characterized in that the physiologically acceptable medium is thickened using thickeners and/or gelling agents and contains preservatives, stabilizers, pH regulators, agents which modify the osmotic pressure, surfactants, UV-A and UV-B filters or antioxidants.

18. Composition according to any one of Claims 7 to 17, for its application as a medicament intended to be used in the therapeutic treatment of hair loss and to stimulate hair growth.

19. Use of the composition as defined in any one of Claims 7 to 17 for the preparation of a medicament intended to be used in the therapeutic treatment of hair loss and to promote hair growth.

## Patentansprüche

1. Verbindung, dadurch **gekennzeichnet,** daß sie der folgenden Formel (I) entspricht: 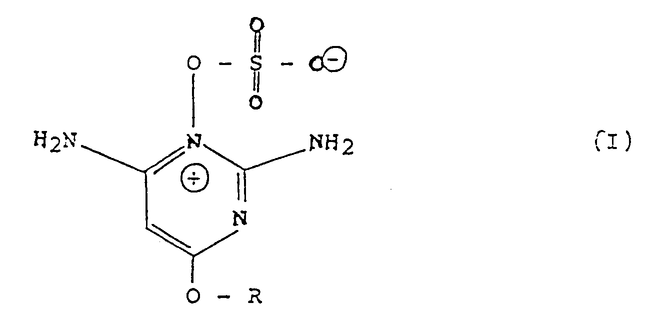 in der R ein lineares oder verzweigtes C₁-C₁₈-Alkylradikal; ein C₂-C₁₈-Alkylenradikal; ein cyclisches, gesättigtes oder ungesättigtes Radikal oder ein C₁-C₆-Alkylradikal, das einen aromatischen oder heterocyclischen Kern hat, bezeichnet.

2. Verbindung nach Anspruch 1, dadurch **gekennzeichnet,** daß der heterocyclische Kern Pyridin ist.

3. Verbindung nach Anspruch 1 oder 2, dadurch **gekennzeichnet**, daß das Radikal R in der Formel (I) aus den Gruppen Ethyl, Butyl, Isobutyl, 3,3-Dimethylbutyl, Octyl, Lauryl, 2-Ethylhexyl, Hexenyl, Undecenyl, Cyclohexyl, Phenetyl oder Benzyl ausgewählt ist.

4. Verbindung nach einem der Ansprüche 1 bis 3, dadurch **gekennzeichnet,** daß die Verbindung der Formel (I) unter den internen Salzen von 2,4-Diamino-6-n-butoxy-3-sulfoxypyrimidinium-Hydroxid; 2,4-Diamino-6-ethoxy-3-sulfoxypyrimidinium-Hydroxid und 2,4-Diamino-6-hexyloxy-3-sulfoxy-pyrimidinium-Hydroxid ausgewählt ist.

5. Verfahren zur Herstellung von Verbindungen nach einem der Ansprüche 1 bis 4, dadurch **gekennzeichnet**, daß man eine Verbindung der Formel (II) 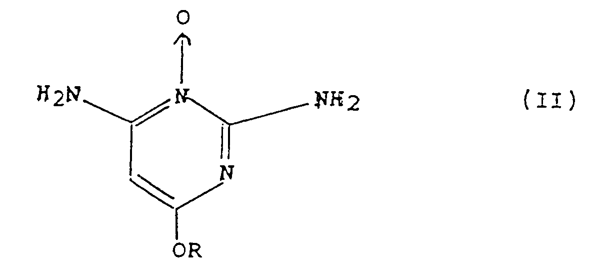 mit einem Derivat, das ein Schwefeltrioxid-(Schwefelsäureanhydrid-)-Entwickler ist, in einem polaren Lösungsmittel bei einer Temperatur zwischen 0°C und 80°C umsetzt.

6. Verfahren nach Anspruch 5, dadurch **gekennzeichnet**, daß das Derivat, das ein Schwefeltrioxid-Entwickler ist, unter den Komplexen von SO₃ und einem tertiären Amin oder einem Gemisch, das in situ aus Chlorsulfonsäure und einem sterisch sperrigen tertiären Amin gebildet wird, ausgewählt ist, und daß das polare Lösungsmittel aus Dimethylformamid oder Chloroform besteht.

7. Zusammensetzung, die dazu bestimmt ist, zum lokalen Auftragen verwendet zu werden, dadurch **gekennzeichnet**, daß sie ein physiologisch akzeptables Medium und mindestens eine Verbindung wie sie in einem der Ansprüche 1 bis 4 definiert ist, enthält.

8. Zusammensetzung nach Anspruch 7, dadurch **gekennzeichnet**, daß sie je nach ihrer pharmazeutischen Anwendung als Salbe, Farbe, Creme, Pomade, Puder, Pflaster, durchtränkter Tampon, Lösung, Emulsion oder bläschenartige Emulsion, Schaum, Lotion, Gel, Spray oder als wasserfreie oder wäßrige Suspension vorliegt, und daß sie mindestens eine Verbindung, wie sie in einem der Ansprüche 1 bis 4 definiert ist, enthält.

9. Zusammensetzung nach Anspruch 7 oder 8, dadurch **gekennzeichnet**, daß die Verbindungen der Formel (I) in Konzentrationen zwischen 0,1 und 10 Gew.%, und insbesondere zwischen 0,2 und 5 Gew.%, bezogen auf das Gesamtgewicht der Zusammensetzung, vorliegen.

10. Zusammensetzung, die zur Verwendung in der Kosmetik bestimmt ist und wie sie im Anspruch 7 definiert ist, dadurch **gekennzeichnet**, daß sie als Lotion, Gel, Creme, Seife, Shampoo, Aerosol oder Schaum vorliegt und daß sie mindestens eine Verbindung, wie sie in einem der Ansprüche 1 bis 4 definiert ist, in einer Konzentration zwischen 0,01 und 3 Gew.% in einem kosmetisch akzeptablen Trägerstoff enthält.

11. Zusammensetzung nach einem der Ansprüche 7 bis 10, dadurch **gekennzeichnet**, daß sie zusätzlich wasseranlagernde Mittel, Mittel gegen Seborrhoe enthält.

12. Zusammensetzung nach einem der Ansprüche 7 bis 11, dadurch **gekennzeichnet,** daß sie auch Mittel enthält, die die Wirksamkeit der Verbindungen der Formel (I) sowohl hinsichtlich eines erneuten Wachsens der Haare wie auch hinsichtlich einer Hemmung des Haarausfalls verbessern.

13. Zusammensetzung nach Anspruch 12, dadurch **gekennzeichnet**, daß die Mittel, die die Wirksamkeit beim erneuten Sprießen der Haare und/oder bei der Hemmung des Haarausfalls verbessern, Nicotinsäureester, steroidische oder nicht-steroidische anti-inflammatorische Agenzien, Retinoide, antibakterielle Agenzien, Calcium-Antagonisten, Hormone, antiandrogene Mittel, Agenzien zum Abfangen von HO-Radikale sind.

14. Zusammensetzung nach Anspruch 12, dadurch **gekennzeichnet**, daß sie Verbindungen, ausgewählt unter Diazoxid; Spiroxason; den Phospholipiden; Linol- und Linolensäure; Salicylsäure und ihren Derivaten; Hydroxycarbonsäuren oder Ketocarbonsäuren, ihren Estern, den Laktonen und ihren entsprechenden Salzen; Anthralin oder 1,8,9-Trihydroxyanthracen; den Carotinoiden; 5,8,11,14-Eikosantetraensäure, 5,8,11-Eikosantriensäure, den Estern und Amiden der beiden letztgenannten Säuren, als Verbindungen zur Verbesserung der Wirksamkeit beim erneuten Wachsen der Haare oder der Hemmung des Haarausfalls enthält.

15. Zusammensetzung nach einem der Ansprüche 7 bis 14, dadurch **gekennzeichnet**, daß das physiologisch akzeptable Medium durch Wasser, ein Gemisch aus Wasser und einem oder mehreren organischen Lösungsmitteln oder ein Gemisch aus organischen Lösungsmitteln gebildet wird, wobei die organischen Lösungsmittel pharmazeutisch oder kosmetisch akzeptabel sind.

16. Zusammensetzung nach Anspruch 15, dadurch **gekennzeichnet**, daß die Lösungsmittel unter den niedrigen C₁-C₄-Alkoholen, den Alkylenglykolen, den Alkylethern von Mono- und Dialkylenglykol ausgewählt sind.

17. Zusammensetzung nach einem der Ansprüche 7 bis 16, dadurch **gekennzeichnet**, daß da physiologisch akzeptable Medium durch Verdickungsmittel und/oder Geliermittel verdickt ist und Konservierungsstoffe, Stabilisatoren, Agenzien zur Regulierung des pH, Modifikatoren für den osmotischen Druck, oberflächenaktive Mittel, UV-A- und UV-B-Filter, Antioxidanzien enthält.

18. Zusammensetzung nach einem der Ansprüche 7 bis 17 zur Anwendung als Arzneimittel, das dazu bestimmt ist, in der therapeutischen Behandlung des Haarausfalls und zur Stimulierung eines erneuten Wachsens verwendet zu werden.

19. Verwendung der Zusammensetzung, wie sie in einem der Ansprüche 7 bis 17 definiert ist, zur Herstellung eines Arzneimittels, das zur therapeutischen Behandlung von Haarausfall und zur Förderung ihres erneuten Wachsens verwendet wird.
